(19) Europäisches Patentamt
European Patent Office
Office européen des brevets

(11) Veröffentlichungsnummer: **0 565 896 A2**

(12) **EUROPÄISCHE PATENTANMELDUNG**

(21) Anmeldenummer: **93104415.0**

(22) Anmeldetag: **18.03.93**

(51) Int. Cl.$^5$: **C07K 5/02**, C07K 5/06, C07K 5/08, C07D 211/34, C07C 279/08, A61K 37/02, A61K 31/445, A61K 31/185

(30) Priorität: **13.04.92 DE 4212304**

(43) Veröffentlichungstag der Anmeldung: **20.10.93 Patentblatt 93/42**

(84) Benannte Vertragsstaaten: **AT BE CH DE DK ES FR GB IT LI NL SE**

(71) Anmelder: **CASSELLA Aktiengesellschaft**
**Hanauer Landstrasse 526**
**D-60386 Frankturt(DE)**

(72) Erfinder: **Klingler, Otmar, Dr.**
**Ernst-Reuter-Strasse 1**
**W-6054 Rodgau(DE)**
Erfinder: **Zoller, Gerhard, Dr.**
**Höhenstrasse 8**
**W-6369 Schöneck(DE)**
Erfinder: **Just, Melitta, Dr.**
**Theodor-Heuss-Strasse 80**
**W-6070 Langen(DE)**
Erfinder: **Jablonka, Bernd, Dr.**
**Dachbergstrasse 19 A**
**W-6232 Bad Soden(DE)**

(74) Vertreter: **Urbach, Hans-Georg, Dr. et al**
**CASSELLA AKTIENGESELLSCHAFT,**
**Patentabteilung,**
**Hanauer Landstrasse 526**
**D-60386 Frankturt (DE)**

(54) **Asparaginsäurederivate, ihre Herstellung und Verwendung.**

(57) Asparaginsäurederivate der Formel

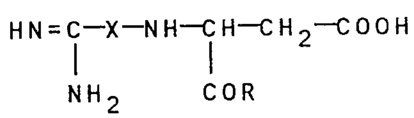

(I)

worin X z.B.

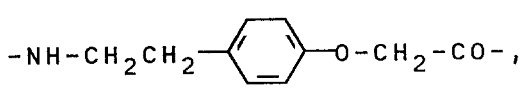

und R z.B. Cyclohexylamino bedeuten, besitzen wertvolle pharmakologische Eigenschaften.

EP 0 565 896 A2

Die Erfindung betrifft Asparaginsäurederivate der allgemeinen Formel I

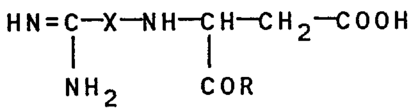

(I)

worin

X

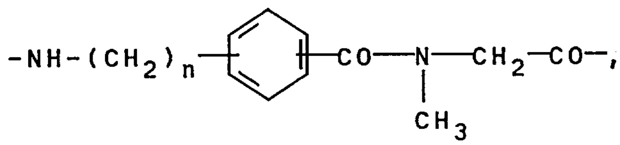

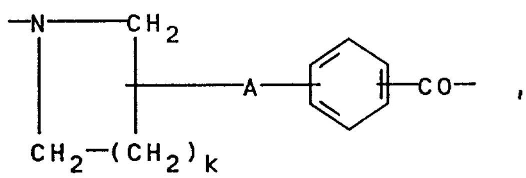

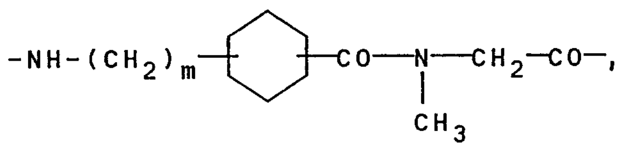

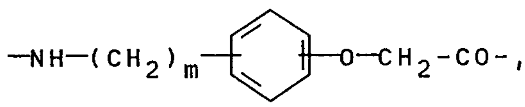

A $-(CH_2)_m-$, -O- oder eine direkte Bindung,

n eine Zahl von 1 bis 4,

k eine Zahl von 0 bis 7,

m eine Zahl von 0 bis 4,

R $-OR^1$,

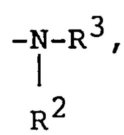

$-NH-R^4$,

$R^1$ Wasserstoff, $C_1$-$C_{28}$Alkyl, $C_3$-$C_{28}$Cycloalkyl, Phenyl, wobei das $C_1$-$C_{28}$Alkyl, das $C_3$-$C_{28}$Cycloalkyl oder das Phenyl unsubstituiert oder ein- oder mehrfach mit gleichen oder verschiedenen Resten aus der Reihe Hydroxy, Carboxy, $C_1$-$C_4$Alkoxycarbonyl, Arylmethoxycarbonyl, Carboxamido, $C_1$-$C_4$Alkylaminocarbonyl, Amino, Mercapto, $C_1$-$C_4$-Alkoxy, $C_3$-$C_8$Cycloalkyl, Imidazolyl, Indolyl, Pyrrolidinyl, Hydroxypyrrolidinyl, Halogen, unsubstituiertem oder ein oder mehrfach mit Hydroxy, $C_1$-$C_4$Alkyl, Halogen, Nitro oder Trifluormethyl, substituiertem Phenyl oder Phenoxy, substituiert ist, ein oder mehrfach durch -O-unterbrochenes $C_3$-$C_{81}$Alkyl oder ein oder mehrfach durch -O- unterbrochenes ($C_3$-$C_{81}$Alkoxy)carbonyl;

$R^2$, $R^3$ unabhängig voneinander Wasserstoff, $R^1$ oder zusammen mit dem Stickstoff, an dem sie gebunden sind, einen 5- oder 6-gliedrigen, gesättigten heterocyclischen Ring, der auch noch -O- oder $-NR^5$- enthalten

kann,
oder $R^2$ Wasserstoff und $R^3$ einen Rest der Formel

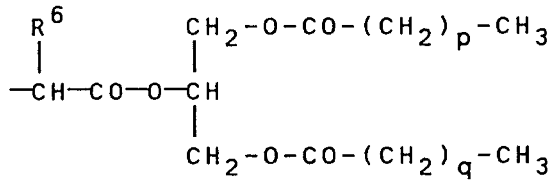

oder

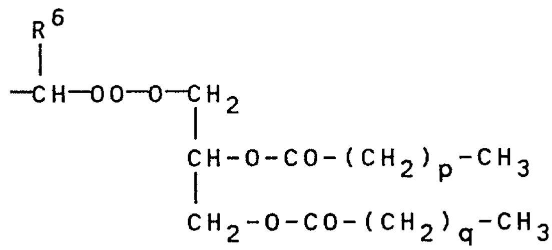

$R^4$ den Rest einer Aminosäure oder eines Dipeptids, bei dem die Peptidbindung auch zu $-NH-CH_2-$ reduziert sein kann,
$R^5$ Wasserstoff, $C_1$-$C_4$ Alkyl, Phenyl, $C_1$-$C_4$ Alkylphenyl, $C_1$-$C_4$ Alkoxyphenyl,
$R^6$ Wasserstoff, $C_1$-$C_4$ Alkyl, Phenyl, Phenyl($C_1$-$C_4$)Alkyl,
p, q unabhängig voneinander eine Zahl von 1 bis 20 bedeuten, sowie deren physiologisch verträglichen Salze.

Die Erfindung betrifft auch die Verfahren zur Herstellung der Verbindungen der Formel I und ihre Verwendung als pharmazeutisch wirksame Stoffe.

Die für $R^1$, $R^2$, $R^3$ und $R^5$ stehenden Alkyl- und Alkoxyreste können geradkettig oder verzweigt sein. Dies gilt auch, wenn sie Substituenten tragen oder als Substituenten anderer Reste auftreten.

Beispiele für geeignete $C_1$-$C_{28}$ Alkylreste sind:
Methyl, Ethyl, Propyl, Butyl, Pentyl, Hexyl, Heptyl, Octyl, Decyl, Dodecyl, Tridecyl, Heptadecyl, Nonadecyl, Eicosyl, Docosyl, Tricosyl, Pentacosyl, Hexacosyl, Heptacosyl, Octacosyl, Isobutyl, Isopentyl, Neopentyl, Isohexyl, 3-Methylpentyl, 2,3,5-Trimethylhexyl, sec-Butyl, tert.-Butyl, tert.-Pentyl, Isohexyl. Unverzweigte $C_1$-$C_{28}$-Alkylreste sind bevorzugt.

Von den $C_3$-$C_{28}$ Cycloalkylresten sind $C_3$-$C_8$ Alkylreste (Cyclopropyl, Cyclobutyl, Cyclopentyl, Cyclohexyl, Cycloheptyl, Cyclooctyl), insbesondere Cyclopentyl und Cyclohexyl, bevorzugt.

Halogen kann z.B. Fluor, Chlor, Brom und Iod sein, von denen Fluor, Chlor und Brom bevorzugt sind.

Das ein- oder mehrfach durch Sauerstoff unterbrochene $C_3$-$C_{81}$ Alkyl enthält bevorzugt die Gruppe

$H_3CO(CH_2CH_2O)_x$,

wobei x eine Zahl von 1 bis 40 ist.

Das ein- oder mehrfach durch Sauerstoff unterbrochene ($C_3$-$C_{81}$ Alkoxy)carbonyl enthält bevorzugt die Gruppe

$H_3CO(CH_2CH_2O)_xCO-$

wobei x eine Zahl von 1 bis 40 ist.

Die $C_3$-$C_8$ Cycloalkylreste können auch insbesondere durch eine Carboxygruppe substituiert sein.

Der Arylrest in dem Arylmethoxycarbonylrest kann z.B. ein $\alpha$- oder $\beta$-Naphthylrest oder insbesondere ein Phenylrest sein.

Ein für $R^1$, $R^2$ oder $R^3$ auftretender Phenyl- oder Phenoxyrest oder ein als Substituent für $R^1$, $R^2$ oder $R^3$ auftretender Phenyl- oder Phenoxyrest kann z.B. ein-, zwei- oder dreifach substituiert sein, sofern dies aus sterischen Gründen oder aus Stabilitätsgründen möglich ist. Bei einer Nitrosubstitution ist in der Regel nur

eine Nitrogruppe, gegebenenfalls neben anderen Substituenten, vorhanden.

Der Imidazolrest ist insbesondere ein 4-Imidazolrest. Der Indolylrest ist insbesondere ein 3-Indolylrest.

Geeignete substituierte Phenylreste sind z.B. 3,5-Dibromo-4-hydroxy-phenyl.

$R^2$ und $R^3$ können auch mit dem Stickstoff, an das sie gebunden sind, einen 5- oder 6-gliedrigen gesättigten, heterocyclischen Ring bilden, der auch noch -O- oder $-N(R^5)-$ enthalten kann. Beispiele für derartige Reste sind 1-Pyrrolidinyl, Piperidino, Morpholino, 1-Piperazinyl, 4-($C_1$-$C_4$Alkyl)-piperazin-1-yl, insbesondere 4-Methyl-piperazin-1-yl, 4-Phenyl-piperazin-1-yl, 4-($C_1$-$C_4$-Alkoxy)-piperazin-1-yl.

Für $R^2$ ist Wasserstoff bevorzugt.

$R^4$ stellt den Rest einer natürlichen oder unnatürlichen Aminosäure oder eines Dipeptids dar, wobei dieser Rest formal durch Abspaltung einer $NH_2$-Gruppe aus der Aminosäure oder dem Dipeptid entstanden ist. Reste von $\beta$-Aminosäuren und insbesondere von $\alpha$-Aminosäuren sind bevorzugt. Die Reste $R^4$ können beispielsweise von folgenden Aminosäuren abgeleitet sein, die falls chiral, in der D- oder L-Form vorliegen können (vgl. Houben-Weyl, Methoden der organischen Chemie, Band XV/1 und 2, Stuttgart, 1974):

Aad, Abu $\gamma$Abu, ABz, 2ABz, $\epsilon$Aca, Ach, Acp, Adpd, Ahb, Aib, $\beta$Aib, Ala, $\beta$Ala, $\Delta$Ala, Alg, All, Ama, Amt, Ape, Apm, Apr, Arg, Asn, Asp, Asu, Aze, Azi, Bai, Bph, Can, Cit, Cys, $(Cys)_2$, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hIle, hLeu, hLys, hMet, hphe, hPro, hSer, hThr, hTrp, hTyr, Hyl, Hyp, 3Hyp, Ile, Ise, Iva, Kyn, Lant, Lcn, Leu, Lsg, Lys, $\beta$Lys, $\Delta$Lys, Met, Mim, Min, nArg, Nle, Nva, Oly, Orn, Pan, Pec, Pen, Phe, Phg, Pic, Pro, $\Delta$Pro, Pse, Pya, Pyr, Pza, Qin, Ros, Sar, Sec, Sem, Ser, Thi, $\beta$Thi, Thr, Thy, Thx, Tia, Tle, Tly, Trp, Trta, Tyr, Val, Tbg, Npg, Chg, Cha, Thia, 2,2-Diphenylaminoessigsäure, 2-(p-Tolyl)-2-phenylaminoessigsäure, 2-(p-Chlorphenyl)-aminoessigsäure.

Der Rest $R^4$ kann auch von einem Dipeptid abgeleitet sein, wobei diese Dipeptide als Bausteine natürliche oder unnatürliche Aminosäuren enthalten können. Ferner können die Reste $R^4$ der natürlichen oder unnatürlichen Aminosäuren und Dipeptide auch als Ester bzw. Amide vorliegen, wie z. B. Methylester, Ethylamid, Semicarbazid oder $\omega$-Amino-($C_4$-$C_8$)-alkylamid.

Funktionelle Gruppen der Aminosäure- und Dipeptid-Reste $R^4$ können geschützt vorliegen. Geeignete Schutzgruppen wie z. B. Urethanschutzgruppen, Carboxylschutzgruppen und Seitenkettenschutzgruppen sind bei Hubbuch, Kontakte (Merck) 1979, Nr. 3, Seiten 14 bis 23 und bei Büllesbach, Kontakte (Merck) 1980, Nr. 1, Seiten 23 bis 35 beschrieben. Insbesondere seien genannt: Aloc, Pyoc, Fmoc, Tcboc, Z, Boc, Ddz, Bpoc, Adoc, Msc, Moc, $Z(NO_2)$, $Z(Hal_n)$, Bobz, Iboc, Adpoc, Mboc, Acm, tert.-Butyl, OBzl, ONbzl, OMbzl, Bzl, Mob, Pic, Trt.

Für R ist $-N(R^2)R^3$ und insbesondere $-NH-R^4$ bevorzugt. Der Rest $-N(R^2)R^3$ bedeutet insbesondere einen Amino-($C_1$-$C_8$)-alkyl-diphenylrest, einen Amino-($C_3$-$C_8$)-cycloalkylrest oder einen Amino-($C_1$-$C_8$)-alkylrest. Der Amino-($C_3$-$C_8$)-cycloalkylrest kann dabei auch am Cycloalkylrest substituiert sein, insbesondere durch eine Carboxygruppe. Der Amino-($C_1$-$C_8$)-alkyl-diphenylrest ist insbesondere ein $\omega$,$\omega$-Diphenylalkylaminrest, ganz besonders bevorzugt ein 3,3-Diphenylpropylaminrest. Besonders bevorzugt steht der für R stehende Rest $-NH-R^4$ für den Valin-, Phenylalanin- oder Phenylglyzinrest, der durch Abspaltung eines Wasserstoffatoms von der Aminogruppe des Valins, Phenylalanins oder Phenylglyzins entsteht.

Für $R^6$ sind Wasserstoff, Phenyl, Benzyl, Methyl oder Isopropyl bevorzugt.

Für k ist 2 und insbesondere 3 bevorzugt. Für p und q ist eine Zahl von 14 bis 16 bevorzugt.

Die für X stehenden Reste enthalten vorzugsweise 1,4- oder 1,3-Phenylen, 1,4-Cyclohexylen, p-Piperidinyl oder 3-Pyrrolidinyl.

Für n ist 1 und für m sind 1 oder 2 bevorzugt.

Besonders bevorzugte Reste X sind:

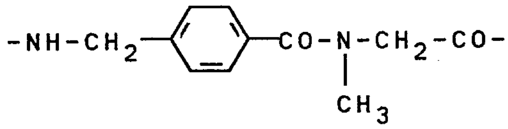,

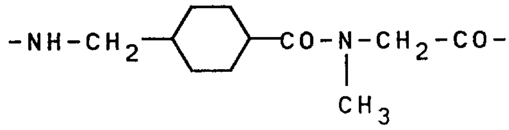,

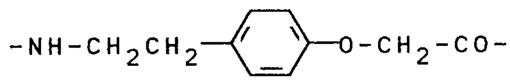,

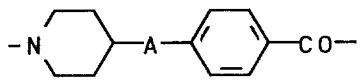,

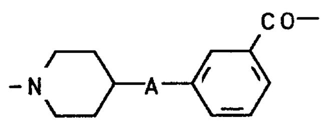,

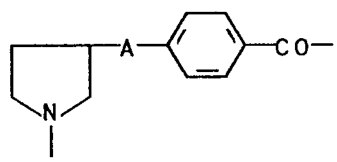,

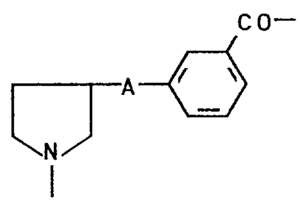,

Bevorzugte Verbindungen der Formel I sind solche, die ein und insbesondere mehrere der bevorzugten Reste oder Gruppen enthalten.

Physiologisch verträgliche Salze der Verbindungen der allgemeinen Formel I sind insbesondere pharmazeutisch verwendbare oder nicht-toxische Salze.

Die Carboxylgruppe der Verbindungen der Formel I kann mit Alkali- oder Erdalkalimetallen, wie z.B. Na, K, Mg und Ca, sowie mit physiologisch verträglichen organischen Aminen, wie z.B. Triethylamin und Tris-(2-hydroxy-ethyl)-amin, Salze bilden.

Verbindungen der allgemeinen Formel I können auch an der basischen Amidinogruppe oder Guanidinogruppe mit anorganischen Säuren und mit organischen Carbon- oder Sulfonsäuren Salze bilden.

Verbindungen der Formel I können dadurch hergestellt werden, daß

a) eine Verbindung der allgemeinen Formel II

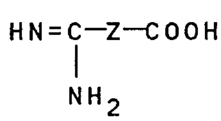 (II)

worin

Z

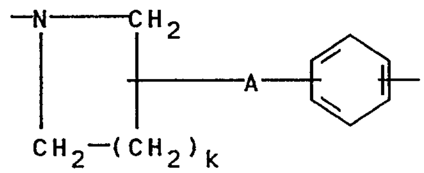

,

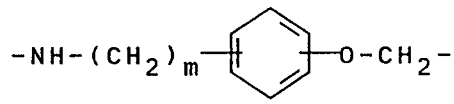

,

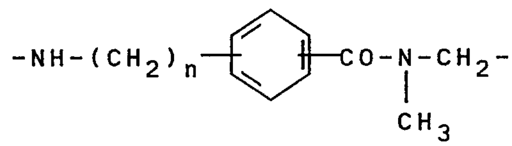

,

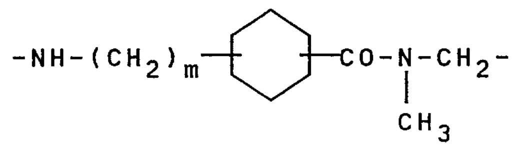

,

bedeutet und A, n und m die bereits angegebenen Bedeutungen besitzen, mit einer Verbindung der allgemeinen Formel III

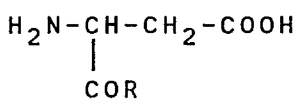

(III)

worin R die bereits genannte Bedeutung besitzt, umgesetzt wird, oder
b) eine Verbindung der allgemeinen Formel IIa

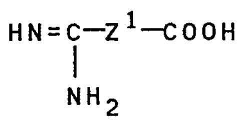

(IIa)

worin
$Z^1$

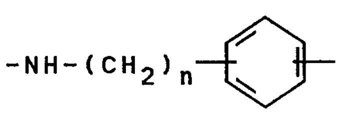

,

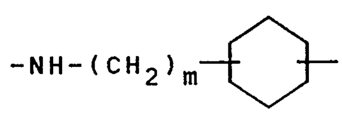

,

bedeutet und n und m die bereits angegebene Bedeutung besitzen, mit einer Verbindung der allgemeinen Formel IIIa

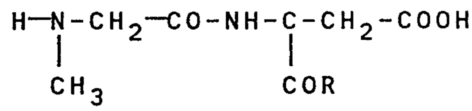 (IIIa)

worin
R die bereits genannte Bedeutung besitzt, umgesetzt wird, oder
c) eine Verbindung der allgemeinen Formel Ia

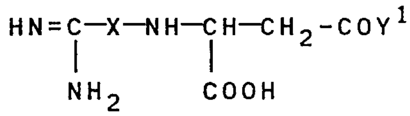 (Ia)

worin X die bereits eingangs genannte Bedeutung besitzt und $Y^1$ eine Schutzgruppe bedeutet, mit einer Verbindung der Formel IV

HR (IV)

worin R die bereits eingangs genannte Bedeutung besitzt, umgesetzt und dann die Schutzgruppe entfernt wird, und daß die Verbindung der Formel I gegebenenfalls in ein physiologisch verträgliches Salz übergeführt wird.

Die Umsetzungen zur Herstellung der erfindungsgemäßen Verbindungen der Formel I stellen im Prinzip Acylierungsreaktionen dar. Sie werden nach bekannten Prinzipien, insbesondere nach den bekannten Methoden der Peptidchemie (vgl. z.B. Houben-Weyl, Methoden der organischen Chemie, Bd. 15/2 (1974); Ullmanns Enzyklopädie der techn. Chemie, 4. Auflage, Bd. 19, S.542-548) durchgeführt. Vorhandene Gruppen, die nicht reagieren sollen, werden durch Schutzgruppen geschützt, die nach der Umsetzung wieder abgespalten werden. Es können alle Schutzgruppen verwendet werden, die unter den Synthesebedingungen stabil sind und nach der Synthese der erfindungsgemäßen Verbindungen wieder abgespalten werden können. Geeignete Aminoschutzgruppen sind z.B. (in Klammer stehen die üblichen Abkürzungen): Benzyloxycarbonyl (Z), tert.Butyloxycarbonyl (Boc), 3,5-Dimethoxyphenylisopropyloxycarbonyl (Ddz), 2-(4-Biphenyl)-iso-propyloxycarbonyl (Bpoc), Trityl (Trt), Methylsulfonyl-ethyloxycarbonyl (Msc), 9-Fluorenylmethylcarbonyl (F-moc).

Guanidinogruppen können z.B. mit $NO_2$- oder mit Methoxytrimethyl-benzolsulfonyl (Mtr)-Gruppen geschützt werden. Als Carboxylschutzgruppen werden z.B. Methyl- oder Ethylester, tert.Butylester, Allyl-, Benzyl- oder Nitrobenzylester verwendet.

Als Schwefelschutzgruppen für im Molekül vorhandene Mercaptoreste sind z.B. Benzyl (Bzl), p-Methoxybenzyl (Mbzl), Trityl (Trt) oder Acetamidomethyl (Acm) geeignet.

Die Bedingungen für die Einführung und Abspaltung der Schutzgruppen sind aus der Peptidchemie bekannt (vgl. z.B. Houben-Weyl loc.cit., Ullmanns loc.cit.)

$NO_2$-Gruppen (Guanidinoschutz), Benzyloxycarbonylgruppen und Benzylestergruppen werden z.B. durch Hydrierung abgespalten. Schutzgruppen vom tert.Butyltyp werden durch saure Hydrolyse abgespalten.

Die Bedingungen für die Knüpfung der Peptidbindung sind ebenfalls aus der Peptidchemie bekannt (vgl. z.B. Houben-Weyl loc.cit., Ullmanns loc.cit.).

Bei der Bildung der Amid- bzw. Peptidbindung ist eine direkte Wasserabspaltung bei der Reaktion der Aminokomponente mit der -COOH-haltigen Komponente durch Zugabe geeigneter Kondensationsmittel möglich, wobei in wasserfreiem Medium gearbeitet wird. Zweckmäßigerweise wird jedoch vor oder während der Umsetzung eine Aktivierung der Carboxylgruppe durchgeführt. Die Aktivierung der Carboxylgruppe kann z.B. nach der Azidmethode oder der Methode der gemischten Anhydride erfolgen. Bei der Methode der gemischten Anhydride werden z.B. Halbester der Kohlensäure verwendet. Besonders bequem ist die Aktivierung der Carboxylgruppe nach der Carbodiimidmethode, z.B. unter Verwendung von Dicyclohexylcarbodiimid (DCC), wobei die Reaktion im Eintopfverfahren durchgeführt werden kann. Zweckmäßigerweise wird die Carbodiimidmethode mit der Aktivestermethode kombiniert, d.h. zusätzlich zu einem Carbodiimid wird eine einen Aktivester bildende N-Hydroxyverbindung, wie z.B. N-Hydroxy-succinimid (HONSu), 1-Hydroxy-benzotriazol (HOBt) oder 3-Hydroxy-4-oxo-3,4-dihydro-1,2,3-benzotriazin (HOObt), eingesetzt. Die Verwendung von HOBt oder HOObt in Kombination mit einem Carbodiimid, insbesondere DCC, ist bevorzugt.

Die Umsetzungen werden zweckmäßigerweise in einem geeigneten inerten Lösungs- oder Dispergiermittel, wie z.B. Wasser, Methanol, Ethanol, Acetonitril, einem Amid, wie Dimethylformamid, Dimethylacetamid, N-Methylpyrrolidon, Methylenchlorid oder einem Ether, wie Tetrahydrofuran, oder einem Gemisch verschiedener Lösungs- oder Dispergiermittel durchgeführt.

Die Umsetzungen können prinzipiell bei Temperaturen zwischen -10°C und dem Siedepunkt des verwendeten Lösungs- oder Dispergiermittels erfolgen. In vielen Fällen wird die Umsetzung bei 0 bis 50°C, insbesondere bei 0 bis 30°C und vorzugsweise bei 0 bis Raumtemperatur, durchgeführt.

Bei der Herstellung der Verbindungen der Formel I werden die Ausgangskomponenten sowie das gegebenenfalls zur Anwendung kommende Aktivierungsmittel oder Aktivierungsmittelgemisch normalerweise in etwa äquimolaren Mengen eingesetzt.

Die erfindungsgemäßen Verbindungen der Formel I bilden mit anorganischen oder organischen Säuren Säureadditionssalze. Zur Bildung derartiger Säureadditionssalze geeignete Säuren sind beispielsweise: Chlorwasserstoff, Bromwasserstoff, Naphthalindisulfonsäuren, insbesondere Naphthalin-1,5-disulfonsäure, Phosphor-, Salpeter-, Schwefel-, Oxal-, Milch-, Glykol-, Sorbin-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Nicotin-, Isonicotin-, Methansulfon-, p-Toluolsulfon-, Zitronen-oder Adipin-Säure. Die Säureadditionssalze werden wie üblich durch Vereinigung der Komponenten, zweckmäßigerweise in einem geeigneten Lösungsmittel oder Verdünnungsmittel, hergestellt.

Carboxylgruppen in den Verbindungen der Formel I können durch Vereinigen in bekannter Weise mit Hydroxiden, wie z.B. Natrium-, Kalium-, Rubidium-, Lithium-, Magnesium-, Calciumhydroxid oder organischen Aminen, Salze bilden.

Die Ausgangsverbindungen der Formel II und IIa können aus den entsprechenden Aminoverbindungen durch Guanylierung oder Nitroguanylierung hergestellt werden. Die Aminoverbindungen sind bekannt bzw. können aus den entsprechenden bekannten Ausgangsverbindungen nach üblichen Methoden hergestellt werden. Für die Guanylierung und Nitroguanylierung können folgende Reagentien verwendet werden:

1. 0-Methylisothioharnstoff (S.Weiss und H.Krommer, Chemiker Zeitung 98 (1974) 617-618);
2. S-Methylisothioharnstoff (R.F.Borne, M.S.Forrester und I.W.Waters, J.Med.Chem. 20 (1977) 771-776);
3. Nitro-S-methylisothioharnstoff (L.S.Hafner und R.E.Evans, J.Org.Chem. 24 (1959) 1157);
4. Formamidinsulfonsäure (K.Kim, Y.-T.Lin und H.S.Mosher, Tetrahedron Lett. 29 (1988) 3183-32186);
5. 3,5-Dimethyl-1-pyrazolyl-formamidinium-nitrat (F.L.Scott, D.G. O'Donovan und J.Reilly, J.Amer.Chem.Soc. 75 (1953) 4053-4054).

Die Ausgangsverbindungen der Formeln III und IIIa werden in bekannter Weise in der Regel vom C-terminalen Ende her stufenweise aus bekannten Verbindungen aufgebaut. Die Peptidknüpfungen können mit den bekannten Kupplungsmethoden der Peptidchemie durchgeführt werden.

Die Verbindungen der Formel III können z.B. in an sich bekannter Weise hergestellt werden durch Umsetzung einer Verbindung der allgemeinen Formel IIIb

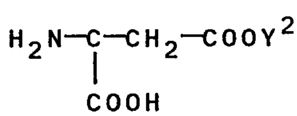

(IIIb)

mit einer Verbindung HR.

Die Verbindungen der Formel IIIa können z.B. in an sich bekannter Weise hergestellt werden durch Umsetzung einer Verbindung der allgemeinen Formel IIIc

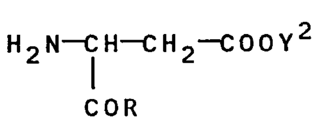

(IIIc)

wobei Y Wasserstoff oder eine geeignete Schutzgruppe bedeutet mit einem in geeigneter Weise geschützten Sarcosin ($HN(CH_3)CH_2COOH$).

Ausgangsverbindungen der Formel Ia werden analog den erfindungsgemäßen Verbindungen aufgebaut. Die Ausgangsverbindungen der Formel IV sind bekannt oder können nach bekannten Methoden hergestellt werden.

Die erfindungsgemäßen neuen Asparaginsäure-Derivate der Formel I und ihre physiologisch verträglichen Salze haben die Fähigkeit, die Bindung von Fibrinogen, Fibronectin und des von Willebrand Faktors an Integrinrezeptoren zu hemmen.

Integrine sind Zellmembran-Glykoproteine und vermitteln Zelladhäsion durch Wechselwirkung mit einer Vielzahl extrazellulärer Proteine, wie Fibronectin, Laminin, Fibrinogen, Kollagen, Vitronectin, und von Willebrand Faktor oder mit anderen Zellmembranproteinen, wie z.B. ICAM-1. Ein wichtiger Rezeptor aus der Integrinfamilie ist das auf Blutplättchen lokalisierte Glykoprotein IIb/IIIa (Fibrinogen-Rezeptor) - ein Schlüsselprotein der Plättchen-Plättchen-Wechselwirkung und Thrombenbildung. Ein zentrales Fragment in der Rezeptorerkennungssequenz dieser Proteine ist das Tripeptid Arg-Gly-Asp (E.Ruoslahti und M.D.Pierschbacher, Science 238 (1987) 491-497; D.R.Phillips, I.F.Charo, L.V.Parise und L.A.Fitzgerald, Blood 71 (1988) 831-843).

Die erfindungsgemaßen Verbindungen der allgemeinen Formel I und ihre physiologisch verträglichen Salze hemmen die Thrombozytenaggregation, die Metastasierung von Karzinomzellen sowie die Osteoclastenbindung an die Knochenoberflächen.

Die Verbindungen der Formel I und ihre physiologisch annehmbaren Salze können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel I oder eines Salzes davon, neben einem oder mehreren üblichen pharmazeutisch einwandfreien Träger-, Füll- oder Verdünnungs- und gegebenenfalls einem oder mehreren Zusatzstoffen enthalten.

Die Heilmittel können oral, z.B. in Form von Tabletten, Filmtabletten, Dragees, Hart- und Weichgelatinekapseln, Mikrokapseln, Granulaten, Pulvern, Pellets, Lösungen, Sirupen, Emulsionen, Suspensionen, Aerosolen, Schäumen, Pillen oder Pastillen verabreicht werden. Die Verabreichung kann aber auch rektal, z.B. in Form von Suppositorien, oder parenteral, z.B. in Form von Injektionslösungen, oder perkutan, z.B. in Form von Salben, Cremes, Gelen, Pasten, Aerosolen, Schäumen, Pudern, Tinkturen, Linimenten oder sogenannten transdermalen therapeutischen Systemen (TTS) oder nasal, z.B. in Form von Nasalsprays, erfolgen.

Die pharmazeutischen Präparate können in an sich bekannter Weise unter Verwendung pharmazeutisch inerter anorganischer oder organischer Hilfs-, Träger-, Füll- oder Verdünnungsstoffe hergestellt werden. Für die Herstellung von Pillen, Tabletten, Filmtabletten, Dragees und die Pellet- oder Granulatfüllungen von Hartgelatinekapseln kann man z.B. Calciumphosphate, Lactose, Sorbitol, Mannitol, Stärken, präparierte Stärken, chemisch modifizierte Stärken, Stärkehydrolysate, Cellulose, Cellulosederivate, synthetische Polymere, Talk etc. verwenden. Träger- oder Verdünnungsstoffe für Weichgelatinekapseln und Suppositorien sind z.B. Fette, Wachse, halbfeste und flüssige Polyole, natürliche oder gehärtete Öle etc. Als Träger- oder Verdünnungsstoffe für die Herstellung von Lösungen und Sirupen eignen sich z.B. Wasser, Polyole, Lösungen von Saccharose, Invertzucker, Glukose etc. Als Trägerstoffe für die Herstellung von Injektionslösungen eignen sich z.B. Wasser, Alkohole, Glyzerol, Polyole oder pflanzliche Öle. Als Träger- oder Verdünnungsstoffe für Salben, Cremes und Pasten eignen sich z.B. Naturvaseline, Kunstvaseline, dick- und dünnflüssige Paraffine, Fette, natürliche oder gehärtete pflanzliche und tierische Öle, Neutralöle, Wachse, Wachsalkohole, Polyethylenglykole, Polyacrylsäure, Silicongele etc. Als Trägerstoffe für Mikrokapseln oder Implantate eignen sich z.B. Mischpolymerisate aus Glykolsäure und Milchsäure.

Die pharmazeutischen Präparate können in an sich bekannter Weise neben den Wirk- und Verdünnungs- oder Trägerstoffen auch noch einen oder mehrere Zusatzstoffe oder Hilfsmittel, wie z.B. Spreng-, Binde-, Gleit-, Netz-, Stabilisierungs-, Emulgier-, Konservierungs-, Süß-, Färbe-, Geschmacks- oder Aromatisierungs-Mittel, Puffersubstanzen, ferner Lösungsmittel oder Lösungsvermittler, Lösungsbeschleuniger, Antischaummittel, Salzbildner, Gelbildner, Verdickungsmittel, Fließregulierungsmittel, Sorptionsmittel, Mittel zur Erzielung eines Depoteffekts oder Mittel, insbesondere Salze, zur Veränderung des osmotischen Drucks, Überzugsmittel oder Antioxidantien etc. enthalten. Sie können auch zwei oder mehrere Verbindungen der Formel I oder ihrer pharmakologisch annehmbaren Säureadditionssalze und noch einen oder mehrere andere therapeutisch wirksame Stoffe enthalten.

Derartige andere therapeutisch wirksame Substanzen sind beispielsweise durchblutungsfördernde Mittel, wie Dihydroergocristin, Nicergolin, Buphenin, Nicotinsäure und ihre Ester, Pyridylcarbinol, Bencyclan, Cinnarizin, Naftidrofuryl, Raubasin und Vincamin; positiv inotrope Verbindungen, wie Digoxin, Acetyldigoxin, Metildigoxin und Lanthano-Glykoside; Coronardilatatoren, wie Carbochromen; Dipyridamol, Nifedipin und Perhexilin; antianginöse Verbindungen, wie Isosorbiddinitrat, Isosorbidmononitrat, Glycerolnitrat, Molsidomin und Verapamil; $\beta$-Blocker, wie Propranolol, Oxprenolol, Atenolol, Metaprolol und Penbutolol. Darüberhinaus

lassen sich die Verbindungen mit anderen nootrop wirksamen Substanzen, wie z.B. Piracetam, oder ZNSaktiven Substanzen, wie Pirlindol, Sulpirid etc. kombinieren.

In den pharmazeutischen Präparaten kann der Gehalt an dem Wirkstoff oder den Wirkstoffen der Formel I in weiten Grenzen schwanken und z.B. 0,05 bis 50 Gew.%, vorzugsweise 0,05 bis 20 Gew.%, betragen. In festen Darreichungsformen, wie Dragees, Tabletten etc. beträgt der Gehalt an einem oder mehreren Wirkstoffen der Formel I in vielen Fällen 2 bis 20 Gew.%. Flüssige Darreichungsformen, wie Tropfen, Emulsionen und Injektionslösungen enthalten häufig 0,05 bis 2 Gew.%, vorzugsweise 0,05 bis 1 Gew.%, eines oder mehrerer Wirkstoffe der Formel I. Der Gehalt an einem oder mehreren Wirkstoffen der Formel I kann gegebenenfalls in den pharmazeutischen Präparaten teilweise, z.B. bis zu 50 Gew.%, vorzugsweise zu 5 bis 40 Gew.%, durch eine oder mehrere andere therapeutisch wirksame Substanzen ersetzt sein.

Die Verbindungen der Formel I, ihre physiologisch annehmbaren Salze und die pharmazeutischen Präparate, welche die Verbindungen der Formel I oder ihre physiologisch annehmbaren Salze als Wirkstoffe enthalten, können am Menschen zur Prophylaxe und Therapie von z.B. arteriellen Gefäßerkrankungen, wie akutem Myokardinfarkt in Kombination mit Lysetherapie, post-Infarktbehandlung, Sekundärprävention des Myokardinfarktes, Reocclusionsprophylaxe nach Lyse und Dilatation, instabiler Angina Pectoris, transitorischer ischämischer Attacken, Schlaganfall, koronarer Bypass-Operation und Reocclusionsprophylaxe des Bypasses, Lungenembolie, peripherer arterieller Verschlußkrankheiten, dissezierendem Aneurysma, zur Therapie venöser und mikrozirkulatorischer Gefäßerkrankungen, wie tiefer Venenthrombose, disseminierter intravaskulärer Gerinnung, post-operativem und post-partum Trauma, chirurgischem oder infektiösem Schock, Septicämie, zur Therapie bei Erkrankungen mit hyperreagiblen Thrombozyten, thrombotische thrombozytopenische Purpura, Preeklampsie, prämenstruelles Syndrom, Dialyse, extrakorporale Zirkulation, ferner bei Entzündungen und bei der Behandlung von Tumoren und der Hemmung der Osteoclastenbindung an die Knochenoberfläche eingesetzt werden.

Die Dosis kann innerhalb weiter Grenzen variieren und ist in jedem einzelnen Fall den individuellen Gegebenheiten anzupassen. Im allgemeinen ist bei der oralen Verabreichung eine Tagesdosis von etwa 0,1 bis 1 mg/kg, vorzugsweise 0,3 bis 0,5 mg/kg Körpergewicht zur Erzielung wirksamer Ergebnisse angemessen, bei intravenöser Applikation beträgt die Tagesdosis im allgemeinen etwa 0,01 bis 0,3 mg/kg, vorzugsweise 0,05 bis 0,1 mg/kg Körpergewicht. Die Tagesdosis wird normalerweise, insbesondere bei der Applikation größerer Mengen, in mehrere, z.B. 2, 3 oder 4 Teilverabreichungen aufgeteilt.

Gegebenenfalls kann es, je nach individuellem Verhalten, erforderlich werden, von der angegebenen Tagesdosis nach oben oder nach unten abzuweichen. Pharmazeutische Präparate enthalten normalerweise 0,2 bis 50 mg, vorzugsweise 0,5 bis 10 mg Wirkstoff der allgemeinen Formel I oder eines ihrer pharmazeutisch akzeptablen Säureadditionssalze pro Dosis.

Geprüft werden die Verbindungen der Formel I vor allem auf ihre hemmende Wirkung bei der Blutplättchenaggregation und der Anhaftung von Fibrinogen an Blutplättchen. Die Messung der Aggregation und der Bindung von $^{125}$I-Fibrinogen wird an gefiltierten, plasmafreien Human-Thrombozyten durchgeführt. Die Aktivierung der Thrombozyten erfolgt durch ADP oder Thrombin.

Als funktioneller Test wird die Hemmung der Aggregation gefiltrierter Human-Thrombozyten nach ADP- oder Thrombin-Stimulierung durch die erfindungsgemäßen Verbindungen gemessen. Angegeben ist der $IC_{50}$-Wert der Hemmung.

Literatur: G.A. Marguerie et al., J. Biol. Chem. 254 (1979), 5357-5363

Angegeben ist der $K_i$-Wert der Bindungshemmung von $^{125}$I-Fibrinogen nach Stimulierung mit ADP (10 µM).

Literatur: J.S. Bennett u. G. Vilaire, J. Clin. Invest. 64, (1979), 1393-1401

E. Kornecki et al., J. Biol. Chem. 256 (1981), 5695-5701

G.A. Marguerie et al., J. Biol. Chem. 254 (1979), 5357-5363

G.A. Marguerie et al., J. Biol. Chem. 255 (1980), 154-161

Bei der Prüfung der Hemmung der Fibrinogenbindung und der Hemmung der Aggregation werden für die Verbindungen der nachfolgenden Beispiele folgende Ergebnisse erhalten:

| Beispiel | Plättchenaggregations-hemmung | | Hemmung der Fibrinogenbindung |
|---|---|---|---|
| | ADP (µM) | Thrombin (µM) | $K_i$ (µM) |
| 1 | 8 | 2 | * |
| 2 | 15 | 10 | * |
| 3 | 15 | 5 | * |
| 4 | 20 | 15 | 10 |
| 9 | 2 | 2 | 1.8 |
| 12 | 8 | 4 | 3.1 |
| 14 | 4.5 | 1.5 | 0.8 |
| *: nicht gemessen | | | |

In den nachstehenden Beispielen werden u.a. folgende Abkürzungen verwendet:

Asp = Asparaginsäure
Val = Valin
Bn = Benzyl
HOBt = 1-Hydroxy-benzotriazol
DMF = Dimethylformamid
DCC = Dicyclohexyl-carbodiimid
DCH = Dicyclohexylharnstoff
Sarc = Sarcosin

**BEISPIELE**

Beispiel 1:

**p-(4-(N-Amidinopiperidinyl)-methyl)-benzoyl-L-aspartyl-L-valin**

a) p-(4-(Nitroamidinopiperidinyl)-methyl)-benzoesäure

Zur Lösung von 4,96 g (22,6 mmol) p-(4-Piperidinylmethyl)-benzoesäure und 1,81 g (45,2 mmol) NaOH in 50 ml Wasser werden bei O°C unter Rühren 3,42 g (25,3 mmol) Nitro-S-methylisothioharnstoff zugesetzt. Nach 24 h Rühren bei Raumtemperatur wird mit konz. HCl auf pH = 1 angesäuert. Der Niederschlag wird abfiltriert, mit Wasser gewaschen und getrocknet.
Ausbeute: 6,13 g (89 %);
Schmelzpunkt: 260 bis 261°C (Zers.).

b) p-(4-(N-Nitroamidinopiperidinyl)-methyl)-benzoyl-Asp(OBn)-Val-OBn

Zur Lösung von 1,11 g (3,64 mmol) p-(4(Nitroamidinopiperidinyl)-methyl)-benzoesäure, 1,50 g (3,64 mmol) $H_2N$-Asp(OBn)-Val-OBn und 0,49 g (3,64 mmol) HOBt in 25 ml DMF gibt man bei 0°C 0,83 g (3,64 mmol) DCC. Nach 4 h Rühren bei 0°C wird weitere 12 h bei Raumtemperatur gerührt. Es wird vom ausgefallenen DCH abfiltriert und der Rückstand chromatographisch gereinigt.
Ausbeute: 2,43 g (95 %), enthält noch etwas DCH;
Schmelzpunkt: 60 bis 75°C.

c) p-(4-(N-Amidinopiperidinyl)-methyl)-benzoyl-L-aspartyl-L-valin

2,29 g (3,27 mmol) p-(4-(N-Nitroamidinopiperidinyl)-methyl)-benzoyl-Asp(OBn)-Val-OBn werden in 20 ml Methanol/10 ml DMF gelöst und mit 0,25 g Pd/C (10 %) versetzt. Nach 6 h Hydrieren bei Raumtemperatur werden 20 ml Wasser und 20 ml Eisessig zugesetzt und weitere 1,5 h hydriert. Vom Katalysator wird abfiltriert, der Rückstand über ®Sephadex LH-20 chromatographiert (Methanol).
Ausbeute: 1,47 g (94 %);
Schmelzpunkt: 260 bis 270°C.
$(\alpha)_D^{20}$ = -5,5° (c = 1 in Eisessig)

Beispiel 2:

**P-(4-(N-Amidinopiperidinyl)-methyl)-benzoyl-L-aspartyl-cyclohexylamid**

a) p-(4-(N-Nitroamidinopiperidinyl)-methyl)-benzoyl-Asp(OBn)-cyclohexylamid

Analog 1b) läßt man 1,51 g (4,93 mmol) p-(4-(Nitroamidinopiperidinyl)-methyl)-benzoesäure mit 1,50 g (4,93 mmol) $H_2N$-Asp(OBn)-cyclohexylamid reagieren.
Ausbeute: 2,3 g (79 %);
Schmelzpunkt: 70 bis 75°C.

b) p-(4-N-Amidinopiperidinyl)-methyl)-benzoyl-L-aspartyl-cyclohexylamid

2,1 g (3,54 mmol) p-(4-N-Nitroamidinopiperidinyl)-methyl)-benzoyl-Asp(OBn)-cyclohexylamid werden analog 1c) hydriert.
Ausbeute: 1,6 g (82 %);
Schmelzpunkt: 220 bis 225°C;
$(\alpha)_D^{20}$ = -5,8° (c = 1,2 in Eisessig)

Beispiel 3:

**p-(4-N-Amidinopiperidinyl)-methyl)-benzoyl-L-aspartyl-3,3-diphenylpropylamid**

a) p-(4-N-Nitroamidinopiperidinyl)-methyl)-benzoyl-Asp(OBn)-3,3-diphenylpropylamid

Analog 1b) läßt man 1,1 g (3,6 mmol) p-(4-(Nitroamidinopiperidinyl)-methyl)-benzoesäure mit 1,50 g (3,6 mmol) $H_2N$-Asp(OBn)-3,3-diphenylpropylamid reagieren.
Ausbeute: 2,15 g (85 %) Acetat;
Schmelzpunkt: 90 bis 100°C.

b) p-(4-N-Amidinopiperidinyl)-methyl)-benzoyl-L-aspartyl-3,3-diphenylpropylamid

1,97 g (2,8 mmol) p-(4-(N-Nitroamidinopiperidinyl)-methy)1-benzoyl-Asp(OBn)-3,3-diphenylpropylamid werden analog 1c) hydriert.
Ausbeute: 1,57 g (89 %) Acetat;
Schmelzpunkt: 160 bis 175°C;
$(\alpha)_D^{20}$ = -6,4° (c = 1,1 in Eisessig)
Analog wurden hergestellt:

Beispiel 4:

**p-(4-N-Amidinopiperidinyl)-methyl)-benzoyl-L-aspartyl-isopropylamid**

Schmelzpunkt: 206 bis 208°C
$(\alpha)_D^{20}$ = +5,6° (c = 1,2 in Eisessig)

Beispiel 5:

**p-(2-Guanidineothyl)-phenoxyacetyl-L-aspartyl-cyclohexylamid**

a) p-(2-Nitroguanidinoethyl)-phenoxyacetyl-L-Asp(OBn)-cyclohexylamid

Analog 1b) läßt man 1,48 g (5,26 mmol) p-(2-Nitroguanidinoethyl)-phenoxyessigsäure mit 1,60 g (5,26 mmol) $H_2N$-Asp(OBn)-cyclohexylamid reagieren.
Ausbeute: 2,51 g (84 %);
Schmelzpunkt: 140 bis 144°C.

b) p-(2-Guanidinoethyl)-phenoxyacetyl-L-aspartyl-cyclohexylamid

1,4 g (2,46 mmol) p-(2-Nitroguanidinoethyl)-phenoxyacetyl-L-Asp(OBn)-cyclohexylamid werden analog 1c) hydriert.
Ausbeute: 0,6 g (56 %);
Schmelzpunkt: 215 bis 219°C;
$(\alpha)_D^{20}$ = +4° (c = 1 in Eisessig)
Analog Beispiel 5 wurden hergestellt:

Beispiel 6:

**p-(2-Guanidinoethyl)-phenoxyacetyl-L-aspartyl-3,3-diphenylpropylamid**

Schmelzpunkt: 194°C (Zers.);
$(\alpha)_D^{20}$ = +3,5° (c = 1,1 in Eisessig)

Beispiel 7:

**p-(2-Guanidinoethyl)-phenoxyacetyl-L-aspartyl-D,L-β-phenyl-β-alaninethylester**

$(\alpha)_D^{20}$ = +9,1° (c = 1 in Eisessig)

Beispiel 8:

p-(2-Guanidinoethyl)-phenoxyacetyl-L-aspartyl-amido-cyclohexan-1-carbonsäure
Schmelzpunkt: 190 bis 199°C;
$(\alpha)_D^{20}$ = -4,8° (c = 1,2 in Eisessig)

Beispiel 9:

**p-Guanidinomethylbenzoyl-sarcosyl-L-aspartyl-3,3-diphenyl-propylamid**

a) p-Nitroguanidinoethylbenzoyl-Sarc-Asp(OBn)-3,3-diphenylpropylamid

Analog 1b) läßt man 0,59 g (2,48 mmol) p-Nitroguanidinomethylbenzoesäure mit 1,21 g (2,48 mmol) $H_2N$-Sarc-Asp-(OBn)-3,3-diphenylpropylamid reagieren.
Ausbeute: 1,37 g (78 %)

b) p-Guanidinomethylbenzoyl-sarcosyl-L-aspartyl-3,3-diphenylpropylamid

1,25 9 (1,77 mmol) p-Nitroguanidinoethylbenzoyl-Sarc-Asp-(OBn)-3,3-diphenylpropylamid werden analog 1c) hydriert. Ausbeute: 0,60 g (54 %)
Schmelzpunkt: 180 bis 195°C;
$(\alpha)_D^{20}$ = -17,6° (c = 1 in Eisessig)

Beispiel 10:

**p-Guanidinomethylbenzoyl-sarcosyl-L-aspartyl-amido-cyclohexan-1-carbonsäure**

Schmelzpunkt: 180 bis 185°C;
$(\alpha)_D^{20}$ = -25,8° (c = 1 in Eisessig)

Beispiel 11:

**trans-4-Guanidinomethylcyclohexancarboxyl-sarcosyl-L-aspartyl-amido-cyclohexan-1-carbonsäure**

a) trans-4-Nitroguanidinomethylcyclohexancarboxyl-Sarc-Asp(OBn)-amido-cyclohexan-1-carbonsäurebenzylester

Analog 1b) läßt man 0,48 g (1,96 mmol) trans-4-Nitroguanidino-methylcyclohexan-1-carbonsäure mit 1,00 g (1,96 mmol) $H_2N$-Sarc-Asp(OBn)-amido-cyclohexan-1-carbonsäurebenzylester reagieren.
Ausbeute: 1,25 g (87 %)

b) trans-4-Guanidinomethylcyclohexancarboxyl-sarcosyl-L-aspartyl-amido-cyclohexan-1-carbonsäure

1,13 g (1,54 mmol) trans-4-Nitroguanidinomethylcyclohexancarboxyl-Sarc-Asp(OBn)-amido-cyclohexan-1-carbonsäurebenzylester werden analog 1c) hydriert. Ausbeute: 0,83 g (94 %);
Schmelzpunkt: 210 bis 220°C;
$(\alpha)_D^{20}$ = -24,6° (c = 1,1 in Eisessig)

Beispiel 12:

**trans-4-Guanidinomethylcyclohexancarboxyl-sarcosyl-L-aspartyl-benzhydrylamid**

$(\alpha)_D^{20}$ = -22,8° (c = 1,1 in Eisessig)

Beispiel 13:

**p-(2-Guanidinoethyl)-phenoxyacetyl-L-aspartyl-D,L-β-phenyl-β-alanin**

a) p-(2-Nitroguanidinoethyl)-phenoxyacetyl-L-Asp(OBn)-D,L-β-phenyl-β-alaninbenzylester

Analog 1b) läßt man 0,92 g (3,26 mmol) p-(2-Nitroguanidinoethyl)-phenoxyessigsäure mit 1,5 g (3,26 mmol) $H_2N$-Asp(OBn)-D,L-β-phenyl-β-alaninbenzylester reagieren.
Ausbeute: 1,28 g (54 %).

b) p-(2-Guanidinoethyl)-phenoxyacetyl-L-aspartyl-D,L-β-phenyl-β-alanin

1,24 g (1,71 mmol) p-(2-Nitroguanidinoethyl)-phenoxyacetyl-L-Asp(OBn)-D,L-β-phenyl-β-alaninbenzylester werden analog 1c) hydriert.
Ausbeute: 0,58 g (68 %);
Schmelzpunkt: 155 bis 175°C
$(\alpha)_D^{20}$ = 0,03° (c = 1 in Eisessig)

Beispiel 14:

**p-(4-N-Amidinopiperidinyloxy)-benzoyl-L-aspartyl-L-valin**

a) p(4-N-Nitroamidinopiperidinyloxy)-benzoyl-L-Asp(OBn)-L-Val-OBn

Analog 1b) läßt man 0,3 g (= 0,97 mmol) p-(4-N-Nitroamidinopiperidinyloxy)-benzoesäure mit 0,4 g (0,97 mmol) $H_2N$-Asp(OBn)-Val-OBn reagieren.
Ausbeute: 0,55 g (81 %).

b) p-(4-N-Amidinopiperidinyloxy-benzoyl-L-aspartyl-L-valin

0,36 g (0,51 mmol) p-(4-N-Nitroamidinopiperidinyloxy)-benzoyl-L-Asp(OBn)-L-Val-OBn werden analog 1c) hydriert.
Ausbeute: 0,23 g (94 %),
Schmelzpunkt: 150°C.

Beispiel 15:

**p-4-N-Amidinopiperidinyl-benzoyl-L-aspartyl-L-valin**

a) p-4-Nitroamidinopiperidinyl-benzoyl-ASp(OBn)-L-Val-OBn

Analog 1b) läßt man 2,5 g (8,55 mmol) p-4-Nitroamidinopiperidinyl-benzoesäure mit 3,56 g (8,55 mmol) $H_2N$-Asp-(OBn)-Val-OBn reagieren.
Ausbeute: 1,92 g (25 %).

b) p-4-N-Amidinopiperidinyl-benzoyl-L-aspartyl-L-valin

Analog 1c) werden 0,42 g (0,61 mmol) p-4-Nitroamidinopiperidinyl-benzoyl-Asp(OBn)-L-Val-OBn hydriert.
Ausbeute: 0,14 g (41 %);
Schmelzpunkt: 190°C (Zers.)

Beispiel 16:

**p-(3-N-Amidinopyrolidinyloxy)-benzoyl-L-aspartyl-L-valin**

a) p-(3-N-Nitroamidinopyrolidinyloxy)-benzoyl)-L-ASp(OBn) -L-Val-OBn

Analog 1b) läßt man 0,29 g (0,99 mmol) p(3-N-Nitroamidinopyrolidinyloxy)-benzoesäure mit 0,41 g (0,99 mmol) $H_2N$-Asp(OBn)-Val-OBn reagieren.
Ausbeute: 0,63 g (92 %).

b) p-(3-N-Amidinopyrolidinyloxy)-benzoyl-L-aspartyl-L-valin

0,63 g (0,92 mmol) p-(3-N-Nitroamidinopyrolidinyloxy)-benzoyl-L-Asp(OBn)-L-Val-OBn werden analog 1c) hydriert. Ausbeute: 0,11 g (27 %);
Schmelzpunkt: 250°C;
$(\alpha)_D^{20}$ = -5,95° (C = 0,84 in Methanol/Wasser 2:1).

Die nachfolgenden Beispiele A bis H betreffen pharmazeutische Zubereitungen.

Beispiel A

Emulsionen mit 3 mg Wirkstoff per 5 ml können nach folgender Rezeptur hergestellt werden:

| | |
|---|---|
| Wirkstoff | 0,06 g |
| Neutralöl | q. s. |
| Natriumcarboxymethylzellulose | 0,6 g |
| Polyoxyethylenstearat | q. s. |
| Reinglycerin | 0,6 bis 2 g |
| Aromastoffe | q. s. |
| Wasser (entmineralisiert oder destilliert) | ad 100 ml |

Beispiel B

Tabletten können nach folgender Formulierung hergestellt werden:

| | |
|---|---|
| Wirkstoff | 2 mg |
| Lactose | 60 mg |
| Maisstärke | 30 mg |
| lösliche Stärke | 4 mg |
| Magnesiumstearat | 4 mg |
| | 100 mg |

Beispiel C

Für die Herstellung von weichgelatinekapseln mit 5 mg Wirkstoff pro Kapsel eignet sich die folgende Zusammensetzung:

| | |
|---|---|
| Wirkstoff | 5 mg |
| Mischung von Triglyceriden aus Kokosöl | 150 mg |
| Kapselinhalt | 155 mg |

Beispiel D

Für die Herstellung von Dragees eignet sich folgende Formulierung:

| | |
|---|---|
| Wirkstoff | 3 mg |
| Maisstärke | 100 mg |
| Lactose | 55 mg |
| sec. Calciumphosphat | 30 mg |
| lösliche Stärke | 3 mg |
| Magnesiumstearat | 5 mg |
| kolloidale Kieselsäure | 4 mg |
| | 200 mg |

Beispiel E

Dragees, enthaltend einen erfindungsgemäßen Wirkstoff und einen anderen therapeutisch wirksamen Stoff:

| | |
|---|---|
| Wirkstoff | 6 mg |
| Propanolol | 40 mg |
| Milchzucker | 90 mg |
| Maisstärke | 90 mg |
| sec. Calciumphosphat | 34 mg |
| lösliche Stärke | 3 mg |
| Magnesiumstearat | 3 mg |
| kolloidale Kieselsäure | 4 mg |
| | 270 mg |

Beispiel F

Dragees, enthaltend einen erfindungsgemäßen Wirkstoff und einen anderen therapeutisch wirksamen Stoff:

| | |
|---|---|
| Wirkstoff | 5 mg |
| Pirlindol | 5 mg |
| Milchzucker | 60 mg |
| Maisstärke | 90 mg |
| sec. Calciumphosphat | 30 mg |
| lösliche Stärke | 3 mg |
| Magnesiumstearat | 3 mg |
| kolloidale Kieselsäure | 4 mg |
| | 200 mg |

Beispiel G

Kapseln, enthaltend einen erfindungsgemäßen Wirkstoff und einen anderen therapeutisch wirksamen Stoff:

| | |
|---|---|
| Wirkstoff | 5 mg |
| Nicergolin | 5 mg |
| Maisstärke | 185 mg |
| | 195 mg |

Beispiel H

Injektionslösungen mit 1 mg Wirkstoff pro ml können nach folgender Rezeptur hergestellt werden:

| | |
|---|---|
| Wirkstoff | 1,0 mg |
| Polyethylenglykol 400 | 0,3 mg |
| Natriumchlorid | 2,7 mg |
| Wasser zu Injektionszwecken auf | 1 ml |

## Patentansprüche

**1.** Asparaginsäurederivate der allgemeinen Formel I

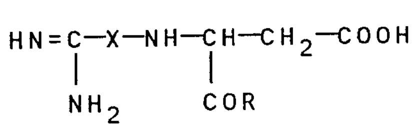

(I)

worin

X

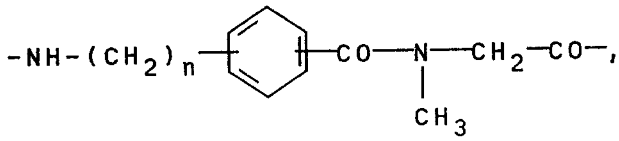

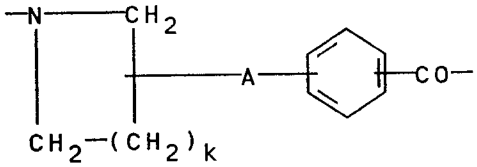

,

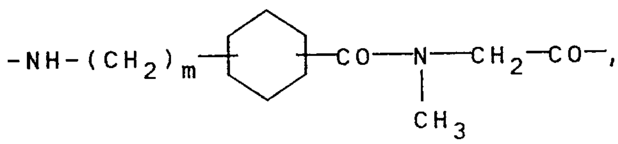

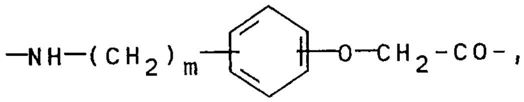

A -$(CH_2)_m$-, -O- oder eine direkte Bindung,
n eine Zahl von 1 bis 4,
k eine Zahl von 0 bis 7,
m eine Zahl von 0 bis 4,
R -$OR^1$,

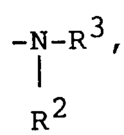

-NH-$R^4$,

$R^1$ Wasserstoff, $C_1$-$C_{28}$Alkyl, $C_3$-$C_{28}$Cycloalkyl, Phenyl, wobei das $C_1$-$C_{28}$Alkyl, das $C_3$-$C_{28}$Cycloalkyl oder das Phenyl unsubstituiert oder ein- oder mehrfach mit gleichen oder verschiedenen Resten aus der Reihe Hydroxy, Carboxy, $C_1$-$C_4$Alkoxycarbonyl, Arylmethoxycarbonyl, Carboxamido, $C_1$-$C_4$Alkylaminocarbonyl, Amino, Mercapto, $C_1$-$C_4$-Alkoxy, $C_3$-$C_8$Cycloalkyl, Imidazolyl, Indolyl, Pyrrolidinyl, Hydroxypyrrolidinyl, Halogen, unsubstituiertem oder ein oder mehrfach mit Hydroxy, $C_1$-$C_4$Alkyl, Halogen, Nitro oder Trifluormethyl, substituiertem Phenyl oder Phenoxy, substituiert ist, ein oder mehrfach durch -O-unterbrochenes $C_3$-$C_{81}$Alkyl oder ein oder mehrfach durch -O- unterbrochenes ($C_3$-$C_{81}$Alkoxy)-carbonyl; $R^2$, $R^3$ unabhängig voneinander Wasserstoff, $R^1$ oder zusammen mit dem Stickstoff, an dem sie gebunden sind, einen 5- oder 6-gliedrigen, gesättigten heterocyclischen Ring, der auch noch -O- oder -$NR^5$- enthalten kann, oder $R^2$ Wasserstoff und $R^3$ einen Rest der Formel

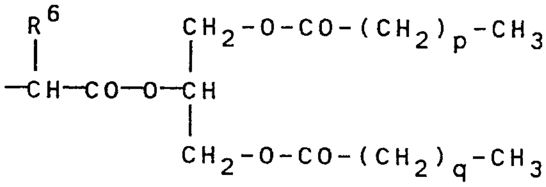

oder

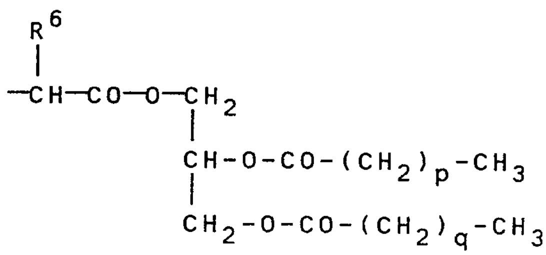

$R^4$ den Rest einer Aminosäure oder eines Dipeptids, bei dem die Peptidbindung auch zu $-NH-CH_2-$ reduziert sein kann,
$R^5$ Wasserstoff, $C_1$-$C_4$ Alkyl, Phenyl, $C_1$-$C_4$ Alkylphenyl, $C_1$-$C_4$ Alkoxyphenyl,
$R^6$ Wasserstoff, $C_1$-$C_4$ Alkyl, Phenyl, Phenyl($C_1$-$C_4$)Alkyl,
p, q unabhängig voneinander eine Zahl von 1 bis 20 bedeuten, sowie deren physiologisch verträglichen Salze.

**2.** Verbindungen nach Anspruch 1, dadurch gekennzeichnet, daß die für X stehenden Reste 1,4- oder 1,3-Phenylen, 1,4-Cyclohexylen, p-Piperidinyl oder 3-Pyrrolidinyl enthalten.

**3.** Verbindungen nach Anspruch 1 und/oder 2, dadurch gekennzeichnet, daß n = 1, k = 2 oder 3 oder m = 1 oder 2 bedeutet.

**4.** Verbindungen nach einem oder mehreren der Ansprüche 1 bis 3, dadurch gekennzeichnet, daß R $-N(R^2)R^3$ oder $-NHR^4$ bedeutet.

**5.** Verbindungen nach einem oder mehreren der Ansprüche 1 bis 4, dadurch gekennzeichnet, daß R $-NHR^4$ bedeutet, wobei $R^4$ für den Valin-, Phenylalanin oder Phenylglyzinrest steht, der formal durch Abspaltung der Aminogruppe aus dem Valin, Phenylalanil oder Phenylglyzin entsteht.

**6.** Verfahren zur Herstellung von Asparaginsäurederivaten eines oder mehrerer der Ansprüche 1 bis 5, dadurch gekennzeichnet, daß
a) eine Verbindung der allgemeinen Formel II

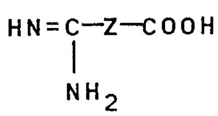 (II)

worin Z

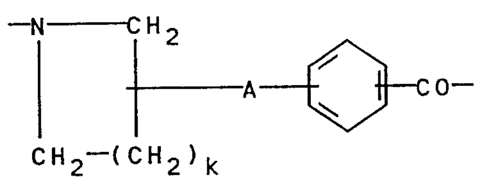 ,

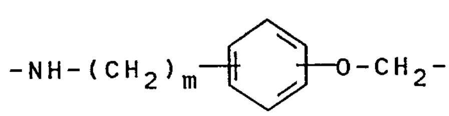 ,

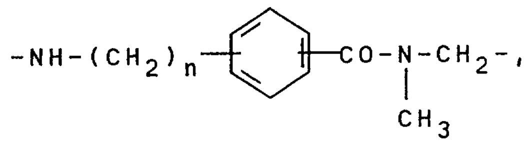

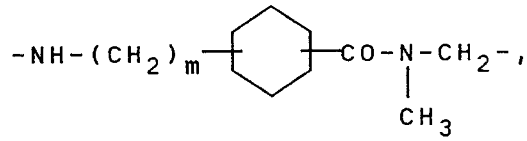

bedeutet und A, n und m die bereits angegebenen Bedeutungen besitzen, mit einer Verbindung der allgemeinen Formel III

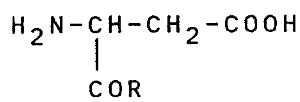

(III)

worin R die bereits genannte Bedeutung besitzt, umgesetzt wird, oder
b) eine Verbindung der allgemeinen Formel IIa

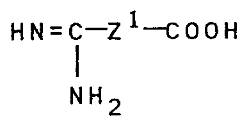

(IIa)

worin $Z^1$

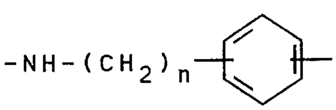 , 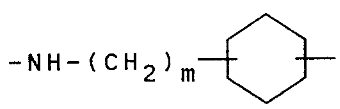 ,

bedeutet und n und m die bereits angegebene Bedeutung besitzen, mit einer Verbindung der allgemeinen Formel IIIa

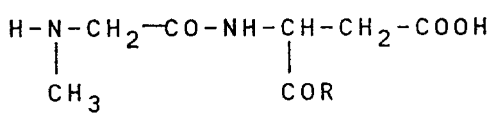

(IIIa)

worin
R die bereits genannte Bedeutung besitzt, umgesetzt wird, oder
c) eine Verbindung der allgemeinen Formel Ia

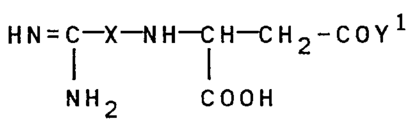

(Ia)

worin X die bereits eingangs genannte Bedeutung besitzt und $Y^1$ eine Schutzgruppe bedeutet, mit einer Verbindung de Formel IV

HR (IV)

worin R die bereits eingangs genannte Bedeutung besitzt, umgesetzt und dann die Schutzgruppe entfernt wird, und daß die Verbindung der Formel I gegebenenfalls in ein physiologisch verträgliches Salz übergeführt wird.

**7.** Verfahren nach Anspruch 7, dadurch gekennzeichnet, daß Aminogruppen, Amidinogruppen, Guanylgruppen, Merkaptogruppen und/oder Carboxylgruppen in den eingesetzten Ausgangsprodukten Schutzgruppen tragen, die gegebenenfalls nach beendeter Umsetzung in an sich bekannter Weise abgespalten werden.

**8.** Verwendung einer Verbindung eines oder mehrerer der Ansprüche 1 bis 5 als Hemmstoffe der Thrombozytenaggregation, der Metastasierung von Karzinomzellen oder der Osteoclastenbildung an die Knochenoberfläche.

**9.** Pharmazeutisches Präparat, dadurch gekennzeichnet, daß es eine oder mehrere Verbindungen der allgemeinen Formel I eines oder mehrerer der Ansprüche 1 bis 5 oder ein physiologisch verträgliches Salz davon als Wirkstoff zusammen mit pharmazeutisch annehmbaren Träger- und Zusatzstoffen und gegebenenfalls noch ein oder mehrere andere pharmakologische Wirkstoffe enthält.

**10.** Verfahren zur Herstellung eines pharmazeutischen Präparates, enthaltend eine oder mehrere Verbindungen der allgemeinen Formel I eines oder mehrerer der Ansprüche 1 bis 5 oder ein physiologisch verträgliches Salz davon, dadurch gekennzeichnet, daß man diese zusammen mit pharmazeutisch annehmbaren Träger- und Zusatzstoffen und gegebenenfalls noch ein oder mehrere andere pharmakologische Wirkstoffe in eine geeignete Darreichungsform bringt.